# EUROPEAN PATENT APPLICATION

(11) **EP 4 800 036 A1**
(43) Date of publication of application: **02.09.2026**
(21) Application number: 23957110.2
(22) Date of filing: 31.10.2023
(51) Int. Cl.: C07K 16/30, C12N 15/13, C12N 15/85, C12N 5/10, C12N 15/62, C07K 19/00, A61K 39/00, A61P 35/00

(54) **ANTIBODY OR TROP2 ANTIGEN BINDING FRAGMENT BINDING TO TROP2, ANTI-TROP2 CAR, AND USE**

(71) Applicant: Shanghai Unicar-Therapy Bio-Medicine Technology Co., Ltd., Shanghai 201203 (CN)
(72) Inventor: KANG, Liqing, Shanghai 201203 (CN); YU, Zhou, Shanghai 201203 (CN)
(74) Representative: Bayramoglu et al.
(86) International application number: PCT/CN2023/128408
(87) International publication number: WO 2025/091232

(57) **Abstract**

The present disclosure discloses a trophoblast cell surface antigen 2 (Trop2)-binding antibody or Trop2 antigen-binding fragment, a nucleic acid encoding the Trop2-binding antibody or Trop2 antigen-binding fragment, a vector and host cell that includes the nucleic acid, an anti-Trop2 chimeric antigen receptor (CAR), and a T cell, a natural killer (NK) cell, a macrophage, a cytokine-induced killer (CIK) cell, and other immune effector cells containing the anti-Trop2 CAR. The present disclosure also discloses a method for producing an antibody or an antigen-binding fragment, and a use of the above-mentioned substances in preparation of an anti-tumor drug, especially in preparation of an anti-tumor drug for the positive expression of Trop2.

## Description

### TECHNICAL FIELD

The present disclosure belongs to the field of biomedicine, and specifically relates to a trophoblast cell surface antigen 2 (Trop2)-binding antibody or Trop2 antigen-binding fragment, an anti-Trop2 chimeric antigen receptor (CAR), and a use thereof.

### BACKGROUND TECHNOLOGY

Globally, malignant tumors are the leading cause of disease-related death and are also a major obstacle to extending human life expectancy. The National Cancer Institute has provided the latest information on the global cancer burden in 2020. In 2020, there were approximately 19.3 million new cancer cases worldwide (18.1 million excluding non-melanoma skin cancer) and nearly 10 million cancer deaths (9.9 million excluding non-melanoma skin cancer). The global cancer burden is projected to reach 28.4 million cases by 2040, representing a 47% increase from 2020. Malignant tumors pose a significant threat to human life and health. For the majority of patients with malignant tumors, the recurrence rate remains high, and the five-year survival rate is consistently low. The main treatment approaches for malignant tumors currently include surgical resection, radiotherapy, chemotherapy, targeted therapy, and immunotherapy. Traditional treatment approaches, such as surgical resection, chemotherapy, and radiotherapy, are generally effective only for patients with early-stage tumors and are associated with significant adverse effects. Targeted drugs, including small molecule inhibitors, offer substantial therapeutic benefits only for patients with specific driver gene mutations. For example, gefitinib is effective only in lung cancer patients harboring epidermal growth factor receptor (EGFR) mutations, and cannot benefit those without EGFR mutations. In recent years, popular cancer immunotherapies, such as monoclonal antibody immune checkpoint inhibitors like anti-PD-L1, anti-PD-1, and anti-CTLA-4, has markedly improved the five-year survival rate in patients with melanoma. However, the therapeutic effect is still not significant for highly malignant solid tumors, such as pancreatic cancer and lung cancer. Therefore, there is an urgent need to develop new treatment modalities to improve the safety and efficacy of treatment for malignant solid tumors.

Chimeric antigen receptor T-cell (CAR-T) therapy is an emerging precision targeted therapy for malignant tumors. In CAR-T therapy, T cells are genetically engineered to allow the expression of CARs including components such as single-chain fragment variables (scFvs) and costimulatory domains on the T cell surface, such that the T cells can recognize specific tumor antigens and thus effectively kill antigen-positive tumor cells. Successful applications of existing CAR-T therapy have been mainly concentrated in the field of hematological malignancies, including B-cell acute lymphoblastic leukemia, large B-cell non-Hodgkin lymphoma, and relapsed/refractory CD19-positive malignant lymphoma. However, CAR-T therapy has so far achieved few breakthroughs in the field of solid tumors. This is mainly because CAR-T therapy currently faces challenges from the following four major aspects in terms of treating solid tumors: 1. Selection of tumor antigens: Most targets for solid tumor therapy, such as Her2, Mesothelin, Muc-1, GPC3, and CEA, are tumor-associated antigens (TAAs). These TAAs are overexpressed on tumor cells relative to normal tissues, but may be expressed on normal tissues at low levels, resulting in potential off-target toxicity. Such potential off-target toxicity significantly limits the clinical application of CAR-T therapy in solid tumors. Therefore, selecting an antigen with a favorable expression profile is crucial for CAR-T therapy. 2. Immunosuppressive tumor microenvironment: In the solid tumor microenvironment, there are numerous inhibitory factors, for example, tumor-associated macrophages (TAMs), regulatory T cells (Tregs), and negative regulatory cytokines such as IL-10 secreted by Tregs, which can promote tumor invasion and migration. Additionally, tumor cells overexpress CD47 and PD-L1, thereby suppressing immune cells. 3. Infiltration of CAR-T cells into the tumor site: The dense extracellular matrix of solid tumors makes it difficult for CAR-T cells to enter the solid tumors and exert their function. 4. Short persistence of CAR-T cells *in vivo*. The challenges of CAR-T therapy in the treatment of solid tumors urgently need to be addressed.

Based on tumor antigen specificity, tumor antigens can be classified into TAAs and tumor-specific antigens (TSAs). TSAs are expressed *only* on tumor cells and are not present on normal cells at different developmental stages. TSAs are generated by the accumulation of genetic mutations in cancer cells (for example, Kras mutations) and exhibit high levels of individual specificity. TAAs are expressed at low levels on normal cells but are expressed at high levels on tumor cells (for example, CD19, CD20, CD38, and Her2).

The present disclosure investigates Trop2, which is TAA and is specifically overexpressed in tumor tissues but expressed at a low level in normal tissues. Trop2, belonging to the tumor-associated calcium signal transducer (TACSTD) family, was initially identified and isolated as a membrane antigen by the monoclonal antibody GA733-1. Accordingly, Trop2 is also referred to as GA733-1. Trop2 is a cell surface glycoprotein encoded by the tumor-associated calcium signal transducer 2 (TACSTD2) gene. Thus, Trop2 is also known as TACSTD2, epithelial glycoprotein 1 (EGP-1), gastrointestinal TAA (GA733-1), and surface marker 1 (M1S1). In general, proteins or peptides are denoted with an initial capital letter followed by lowercase letters (such as Trop2), whereas genes or nucleotide fragments are denoted in all uppercase letters (such as Trop2). Trop2, which is overexpressed in various malignant tumors, is an oncogene associated with the occurrence, invasion, and metastasis of malignant tumors. Trop2 is expressed at low levels in most normal tissues but is overexpressed in a variety of malignant tumors.

Currently, Immunomedics and Daiichi Sankyo are conducting research targeting Trop2, both of which have achieved relatively rapid progress. The treatment of non-small cell lung cancer (NSCLC) by the antibody-drug conjugate (ADC) Ds-1062 from Daiichi Sankyo has entered Phase I clinical trials. In July 2020, Daiichi Sankyo also reached a global development and commercialization licensing agreement with AstraZeneca for Ds-1062, with a total value of up to $6 billion. The ADC Trodelvy from Immunomedics was approved for marketing in April 2020 to treat triple-negative breast cancer. Trodelvy also demonstrates favorable efficacy in patients with advanced NSCLC, with overall survival extending to 9.5 months. In China, Everest Medicines entered into an exclusive licensing agreement with Immunomedics in 2019 and thus acquired the rights to develop, register, and commercialize the marketed ADC product IMMU-132 from Immunomedics in Greater China, South Korea, and several Southeast Asian countries, with a transaction value of up to $835 million. In April 2020, Everest Medicines received approval for the clinical trial application of IMMU-132 in China and planned to initiate clinical research and development programs for various solid tumor indications. In 2021, Henlius was granted an exclusive license from Chiome to research, develop, manufacture, and commercialize antibodies targeting human Trop2, committing over $100 million to advance anti-Trop2 monoclonal antibody development. The ADC SKB-264 from Sichuan Kelun Pharmaceutical Co., Ltd. was approved for clinical trials in the treatment of solid tumors in 2019 and has now advanced to Phase II clinical trials. BAT8003 from Bio-Thera Solutions is in Phase I clinical trials for the treatment of gastric cancer and breast cancer. DAC-002 from Hangzhou DAC Biotechnology Co., Ltd. has been approved for clinical trials. Currently, most drugs targeting Trop2 are ADCs.

The Trop2-targeting ADC drug Trodelvy (Sacituzumab govitecan, IMMU-132) of Immunomedics was approved for marketing in April 2020 for the treatment of triple-negative breast cancer, making it the world's first approved ADC targeting human Trop2. In March 2020, updated clinical data for IMMU-132 in the treatment of triple-negative breast cancer were released. As a third-line treatment, IMMU-132 enabled a progression-free survival (PFS) rate of 33.3% and a disease control rate of 45%, and 74% of patients receiving IMMU-132 experienced tumor shrinkage at least once. IMMU-132 not only demonstrates remarkable efficacy against breast cancer but also improves the overall response rate in patients with lung cancer. In August 2017, Rebecca Suk Heist et al. reported the clinical trial results of IMMU-132 in 44 patients with NSCLC, showing an objective response rate (ORR) of 19%, a clinical benefit rate (complete response + partial response + stable disease ≥ 4 months) of 43%, and PFS of 5.2 months. In May 2017, Jhanelle E. Gray et al. reported the clinical trial results of IMMU-132 in 50 patients with small cell lung cancer, showing ORR of 14%, a clinical benefit rate (complete response + partial response + stable disease ≥ 4 months) of 34%, and PFS of 3.7 months. IMMU-132 exhibits favorable clinical outcomes against NSCLC.

As described above, numerous Trop2-targeting ADC drugs have entered clinical research or have been approved for marketing. However, research on CAR-modified immune cells targeting Trop2 is still in the exploratory stage due to the challenges faced by CAR-T therapy in treating solid tumors as previously described.

### CONTENT OF THE INVENTION

To address the first challenge faced by CAR-T therapy in treating solid tumors, namely, identification of TAAs with a broad expression profile, in a first aspect, the present disclosure provides a Trop2-binding antibody or Trop2 antigen-binding fragment, including:
a1) a heavy-chain variable region (VH) including VH complementarity-determining region (CDR)1 of SEQ ID NO. 3, VH CDR2 of SEQ ID NO. 4, and VH CDR3 of SEQ ID NO. 5; and a light-chain variable region (VL) including VL CDR1 of SEQ ID NO. 6, VL CDR2 of a tripeptide sequence Lys-Ala-Ser, and VL CDR3 of SEQ ID NO. 7; or
a2) a heavy-chain variable region including VH CDR1 of SEQ ID NO. 8, VH CDR2 of SEQ ID NO. 9, and VH CDR3 of SEQ ID NO. 10; and a light-chain variable region including VL CDR1 of SEQ ID NO. 11, VL CDR2 of SEQ ID NO. 12, and VL CDR3 of SEQ ID NO. 13; or
a3) a heavy-chain variable region including VH CDR1 of SEQ ID NO. 14, VH CDR2 of SEQ ID NO. 15, and VH CDR3 of SEQ ID NO. 10; and a light-chain variable region including VL CDR1 of SEQ ID NO. 11, VL CDR2 of SEQ ID NO. 12, and VL CDR3 of SEQ ID NO. 13;
the Trop2-binding antibody is a polyclonal antibody or an anti-Trop2 monoclonal antibody; and
the Trop2 antigen-binding fragment is:
   b1) a scFv; or
   b2) a disulfide-stabilized Fv; or
   b3) a fragment antigen-binding (Fab); or
   b4) a fragment antigen-binding prime (F(ab')); or
   b5) a monovalent fragment consisting of a VL domain, a VH domain, a light-chain constant region (CL) domain, and a heavy-chain constant region 1 (CH1) domain; or
   b6) a fragment antigen-binding prime 2 (F(ab')₂), which is a bivalent fragment including two Fab fragments linked by a disulfide bond in the hinge region; or
   b7) a Fv fragment consisting of the VL domain and a VH domain of a single arm of an antibody.

In some embodiments, the Trop2-binding antibody or Trop2 antigen-binding fragment includes: a heavy-chain variable region including an amino acid sequence of SEQ ID NO. 1 and a light-chain variable region including an amino acid sequence of SEQ ID NO. 2.

In some embodiments, the Trop2-binding antibody or Trop2 antigen-binding fragment is a single-chain antibody.

In some embodiments, the Trop2-binding antibody is a human antibody, a murine antibody, or a camelid antibody.

In some embodiments, the Trop2-binding antibody or Trop2 antigen-binding fragment is a fully human antibody or a fully human antigen-binding fragment.

In some embodiments, the heavy-chain variable region and the light-chain variable region are linked by a (G4S)3 linker.

In some embodiments, an amino acid sequence of a heavy-chain variable region VH of the Trop2-binding antibody is shown in SEQ ID NO. 1; an amino acid sequence of a heavy-chain constant region CH1 of the Trop2-binding antibody is shown in SEQ ID NO. 16; an amino acid sequence of a heavy-chain constant region CH2 of the Trop2-binding antibody is shown in SEQ ID NO. 17; and an amino acid sequence of a heavy-chain constant region CH3 of the Trop2-binding antibody is shown in SEQ ID NO. 18.

In some embodiments, an amino acid sequence of a light-chain variable region VL of the Trop2-binding antibody is shown in SEQ ID NO. 2; and an amino acid sequence of a light-chain constant region CL of the Trop2-binding antibody is shown in SEQ ID NO. 19.

In a second aspect, the present disclosure further provides a nucleic acid encoding the Trop2-binding antibody or Trop2 antigen-binding fragment.

In some embodiments, the nucleic acid includes a nucleotide sequence encoding the heavy-chain variable region including SEQ ID NO. 20 and a nucleotide sequence encoding the light-chain variable region including SEQ ID NO. 21.

In a third aspect, the present disclosure further provides a vector including the nucleic acid.

In some embodiments, the vector is a recombinant lentiviral vector; the recombinant lentiviral vector contains a nucleotide sequence of anti-Trop2 scFv; the anti-Trop2 scFv is the Trop2 antigen-binding fragment; and the Trop2 antigen-binding fragment is scFv.

In a fourth aspect, the present disclosure further provides a host cell including the nucleic acid or the vector.

In a fifth aspect, the present disclosure further provides a method for producing an antibody or an antigen-binding fragment, including: culturing the host cell and recovering the antibody or the antigen-binding fragment from a resulting culture.

In a sixth aspect, the present disclosure further provides anti-Trop2 CAR including anti-Trop2 scFv; the anti-Trop2 scFv is the Trop2 antigen-binding fragment according to claim 1; and the Trop2 antigen-binding fragment is scFv.

In some embodiments, a structure of the anti-Trop2 CAR is cluster of differentiation (CD)8 leader - anti-Trop2 scFv - CD8 Hinge - CD8 transmembrane domain (TM) - costimulatory domain - intracellular signaling peptide, including a CD8 leader membrane receptor signal peptide, anti-Trop2 scFv, a CD8 Hinge chimeric receptor hinge region, a CD8 TM chimeric receptor transmembrane region, a costimulatory domain, and an intracellular signaling peptide that are linked in tandem sequentially.

In some embodiments, the costimulatory domain includes, but is not limited to, one or more selected from the group consisting of CD28, OX40, and 4-1BB. Further, the intracellular signaling peptide is CD3ζ.

In some embodiments, the structure of the CAR includes, but is not limited to, the CD8 leader - anti-Trop2 scFv - CD8 Hinge - CD8 TM - costimulatory domain - intracellular signaling peptide.

In a seventh aspect, the present disclosure further provides an anti-Trop2 CAR-T, anti-Trop2 chimeric antigen receptor natural killer cell (CAR-NK), or anti-Trop2 chimeric antigen receptor macrophage (CAR-M), which are modified with CAR, wherein the CAR is the anti-Trop2 CAR.

In some embodiments, a T cell used for the anti-Trop2 CAR-T includes, but is not limited to, one or more selected from the group consisting of a αβ T cell, a γδ T cell, a natural killer T (NKT) cell, a mucosal-associated invariant T (MAIT) cell, and a cytokine-induced killer (CIK) cell.

In an eighth aspect, the present disclosure further provides a use of the Trop2-binding antibody or Trop2 antigen-binding fragment or the nucleic acid or the vector or the host cell or the anti-Trop2 CAR or the anti-Trop2 CAR-T, anti-Trop2 CAR-NK, or anti-Trop2 CAR-M in preparation of an anti-tumor drug.

In some embodiments, the tumor is capable of expressing Trop2.

In some embodiments, the tumor is a solid tumor that positively expresses Trop2.

In some embodiments, the tumor is capable of expressing Trop2; the tumor includes, but is not limited to, one or more selected from the group consisting of pancreatic cancer, gastric cancer, colorectal cancer, breast cancer, prostate cancer, lung cancer, and oral squamous cell carcinoma.

In some embodiments, the breast cancer includes triple-negative breast cancer, and the lung cancer includes small cell lung cancer and NSCLC.

As used herein, unless specifically indicated as an antibody from a particular source in specific embodiments, the term "Trop2-binding antibody" shall be understood to encompass antibodies from other sources, for example, it can be human antibodies, humanized antibodies, murine antibodies, or antibodies from other sources.

As used herein, unless specifically indicated as a particular type of T cells in specific embodiments, T cells involved in the term "anti-Trop2 CAR-T" shall be understood as T cells in a broad sense, including, but not limited to, αβ T cells and/or γδ T cells, and further include atypical T cells, such as NKT cells, MAIT cells, and CIK cells. These atypical T cells also serve as effector types of T lymphocytes. A source of the T cells is also not limited to peripheral blood. The sources of T cells are not limited to peripheral blood; they also include cells derived from differentiation, induction, and transformation of umbilical cord blood, stem cells, iPSCs, cell lines, etc., as well as other cell types differentiated from T cells. The αβ T cells and γδ T cells are classifications of typical T cells based on different T cell receptor (TCR) types.

As used herein, the term "host cell" shall be understood in a broad sense and may be a prokaryotic cell or a eukaryotic cell. The host cell may be any suitable host cell known in the art, for example, a mammalian host cell, a bacterial host cell, a yeast host cell, and an insect host cell.

As used herein, the term "fully human antibody" refers to an antibody in which both constant regions and variable regions are derived from humans (0% of murine origin and 100% of human origin). The fully human antibody can be produced by technologies such as phage display, yeast display, ribosome display, or single-cell polymerase chain reaction (PCR) fully human monoclonal antibody technology. The anti-Trop2 antibody in the specific embodiments herein is a fully human antibody screened from a fully human scFv antibody library by phage display technology.

As used herein, the term "monoclonal antibody", "mAb" or "immunoglobulin G (IgG)" consists of a heavy chain and a light chain. The light chain consists of a light-chain variable region (VL) and a light-chain constant region (CL); the heavy chain consists of a heavy-chain variable region (VH), a heavy-chain constant region 1 (CH1), a heavy-chain constant region 2 (CH2), and a heavy-chain constant region 3 (CH3), and can specifically bind to an antigen. It is a heterotetrameric glycoproteins that have a molecular weight of approximately 150,000 Daltons and share common structural features. Each light chain is linked to a heavy chain by a covalent disulfide bond. The number of disulfide bonds between heavy chains varies among different immunoglobulin (Ig) isotypes. Each heavy or light chain also has regularly spaced intrachain disulfide bonds. Each heavy chain has a variable region (VH) at one end, followed by a constant region. Each light chain has a variable region (VL) at one end and a constant region at the other end. Special amino acid residues form an interface between variable regions of a light chain and a heavy chain.

As used herein, the term "variable region" or "V region" refers to a region with diverse amino acid compositions and sequences. Both the heavy-chain variable regions and light-chain variable regions include three complementarity determining regions (CDR1, CDR2, and CDR3) and four framework regions (FR1, FR2, FR3, and FR4).

As used herein, the term "complementarity determining region" or "CDR" refers to a highly variable region that participates in the formation of an antigen-binding site.

As used herein, the term "constant region" or "C region" refers to a region with a relatively stable amino acid composition and sequence. Constant regions of a heavy chain and a light chain are referred to as CH and CL, respectively.

As used herein, the term "antibody heavy chain" consists of a heavy-chain variable region (VH), a heavy-chain constant region 1 (CH1), a heavy-chain constant region 2 (CH2), and a heavy-chain constant region 3 (CH3) and can bind to an antigen.

As used herein, the term "antibody light chain" consists of a light-chain variable region (VH) and a light-chain constant region (CL) and can bind to an antigen. Based on the antigenic specificity of a constant region of a light chain of Ig, the light chain can be classified into the κ or λ type. The differences in antigenic specificity determining an Ig type arise from different amino acid compositions, sequences, and spatial conformations of light-chain constant regions (CLs). Accordingly, a light chain is classified into the κ or λ type. The anti-Trop2 monoclonal antibody in the present disclosure has a κ-type light chain.

As used herein, the term "single-chain fragment variable (scFv)" consists of a light-chain variable region (VL), a heavy-chain variable region (VH), and a linker and can bind to an antigen.

As used herein, the term "fragment crystallizable (Fc) region" consists of CH2 and CH3 domains of IgG and a hinge region.

As used herein, the terms "treatment", "therapy", and "therapeutic intervention" can be used interchangeably. The term "treatment" includes controlling the progression of a disease, disorder, or condition and associated symptoms, and preferably includes alleviating a disease, disorder, or condition or reducing the impact of one or more symptoms of the disease, disorder, or condition. The terms include curing a disease or completely eliminating symptoms. The terms include the alleviation of symptoms. The terms also include, but are not limited to, non-curative palliative treatment. The term "treatment" includes administering a therapeutically effective amount of a pharmaceutical composition including the recombinant protein or fusion protein of the present disclosure to a subject, so as to prevent or delay, alleviate or relieve the progression of a disease, disorder, or condition or the impact of one or more symptoms of the disease, disorder, or condition.

As used herein, the term "NCG mice" (NOD/ShiLtJGpt-Prkdc em26Il2rg em26/Gpt, strain type: KnoCL out, strain number: T001475, background: NOD/ShiltJGpt) refers to mice lacking mature T, B, and NK cells, which serve as an important carrier for humanized mice, xenotransplantation, and immune reconstitution. The NCG mice are of great significance for research on human hematopoietic stem cells, tumorigenesis and tumor treatment, immunodeficiency disorders, and *in vivo* immune mechanisms. Due to the lack of animal models, the current studies in the CAR therapy field mostly employ immunodeficient mice for *in vivo* experiments.

Through flow cytometry analysis of multiple solid tumor cell lines, the present disclosure reveals that Trop2 is overexpressed in various malignant solid tumor cell lines and can serve as a novel target for the treatment of solid tumors. The Trop2-binding antibody, the anti-Trop2 scFv, and other Trop2 antigen-binding fragments disclosed in the present disclosure can specifically bind to Trop2, thereby exerting a cell-killing effect.

Compared with Trop2-targeting ADC drugs, the anti-Trop2 CAR-modified immune cells employed by the present disclosure offer the following advantages: CAR-T cell, as a living drug, can proliferate *in vivo* upon receiving antigen stimulation, resulting in great persistence and long duration of action. After being stimulated by target cells, CAR-T cells can release various cytotoxic molecules such as granzymes and perforin, as well as pro-inflammatory cytokines such as interferon-gamma (IFN-γ). T cells express a variety of chemokine receptors on the surface, such as CCR2 and CCR4, which can exhibit chemotaxis toward tumor tissues to provide enhanced targetability. Further, since T cells of a patient himself/herself are collected, genetically engineered, and then reinfused into the patient, the immune rejection is avoided in the patient. In the absence of antigen stimulation *in vivo*, CAR-T cells do not exert their function. In contrast, because highly active cytotoxic drugs are conjugated in ADC drugs, these cytotoxic drugs may cause severe toxic and side effects if released at non-tumor sites. Therefore, the anti-Trop2 CAR-modified immune cells offer high safety. Moreover, compared with CAR-T cells, ADC drugs have a short half-life *in vivo*, making patients prone to relapse.

### DESCRIPTION OF THE DRAWINGS

To describe the technical solutions in the specific implementations of the present disclosure or the prior art clearly, the accompanying drawings required for describing the specific implementations or the prior art are briefly described below. Apparently, the accompanying drawings in the following description show merely some implementations of the present disclosure, and a person of ordinary skill in the art may still derive other accompanying drawings from these accompanying drawings without creative efforts.
FIG. 1 shows OD₄₅₀ readings of anti-Trop2 phage antibodies screened by Phage Enzyme-Linked Immunosorbent Assay (Phage ELISA).
FIG. 2 shows Sodium Dodecyl Sulfate-Polyacrylamide Gel Electrophoresis (SDS-PAGE) results of a purified T3 monoclonal antibody, wherein two lanes are: M-Marker and T3-anti-Trop2 monoclonal antibody, respectively.
FIG. 3 shows the affinity of an anti-Trop2 antibody for an antigen as determined by Enzyme-Linked Immunosorbent Assay (ELISA), wherein NC is a negative control, which is an unrelated antibody that does not bind to the Trop2-His antigen.
FIG. 4 shows flow cytometry identification results of the binding of an antibody to cell surface antigens, wherein T3 refers to cells to be tested are co-incubated with a T3 monoclonal antibody; and blank refers to cells to be tested without any antibody, which serve as a blank control.
FIG. 5 shows schematic diagrams of structures of CARs in anti-Trop2 CAR-T cells and anti-CD19 CAR-T cells in a specific embodiment, wherein a is for anti-Trop2 CAR and b is for anti-CD19 CAR.
FIG. 6 shows transduction efficiencies of D7 and D17 CAR-T cells as measured by FITC-Protein L detection, wherein in coordinate axes, SSC-H represents a side scatter channel and CAR-FITC-H represents an FITC channel.
FIG. 7 shows lactate dehydrogenase (LDH)-based cytotoxicity of anti-Trop2 CAR-T cells against different solid tumor cell lines *in vitro*.

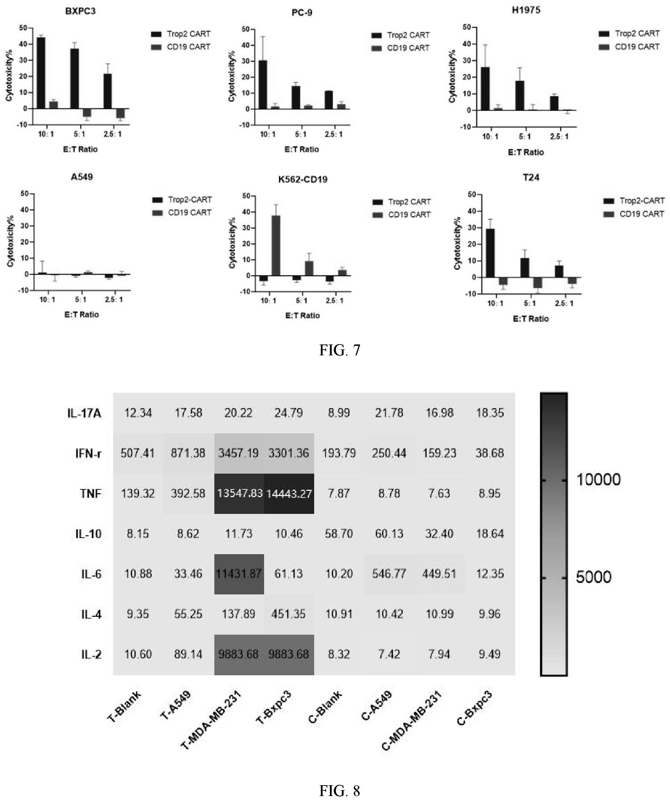

FIG. 8 shows cytokine release levels of anti-Trop2 CAR-T cells co-incubated with different solid tumor cell lines *in vitro*, where T-Blank refers to blank anti-Trop2 CAR-T cells without target cells; T-A549 refers to anti-Trop2 CAR-T cells co-incubated with A549 cells (Trop2 expression negative); T-MDA-MB-231 refers to anti-Trop2 CAR-T cells co-incubated with MDA-MB-231 cells (Trop2 expression positive); T-Bxpc3 refers to anti-Trop2 CAR-T cells co-incubated with Bxpc3 cells (Trop2 expression positive); C-Blank refers to blank anti-CD19 CAR-T cells without target cells; C-A549 refers to anti-CD19 CAR-T cells co-incubated with A549 cells (Trop2 expression negative); C-MDA-MB-231 refers to anti-CD19 CAR-T cells co-incubated with MDA-MB-231 cells (Trop2 expression positive); and C-Bxpc3 refers to anti-CD19 CAR-T cells co-incubated with Bxpc3 cells (Trop2 expression positive); a co-incubation ratio of effector cells to target cells is 5:1; and after co-incubation for 24 h, a resulting culture supernatant is collected and tested for cytokine levels using a CBA kit by flow cytometry.
FIG. 9 shows the specific killing of MDA-MB-231 triple-negative breast cancer cells *in vivo* and significant inhibition of tumor growth by anti-Trop2 CAR-T cells, wherein a shows tumor volume curves and b shows mouse body weight change curves.
FIG. 10 shows the specific killing of H1975 lung cancer cells *in vivo* and significant inhibition of tumor growth by anti-Trop2 CAR-T cells, wherein a shows tumor volume curves and b shows mouse body weight change curves.

### SPECIFIC IMPLEMENTATIONS

To facilitate understanding of the present disclosure, the present disclosure will be described with reference to some embodiments and in specific language. However, it should be understood that these specific embodiments are not intended to limit the scope of the present disclosure. Any changes and further modifications made to the embodiments and any further applications of the present disclosure will be apparent to those skilled in the art.

Nucleotide sequence and amino acid sequence information involved in the present disclosure is set forth in the accompanying tables.

### Description of the accompanying tables

**Table 1 Amino acid sequences of light-chain and heavy-chain variable regions of a T3-positive clone screened by phage display**

| Sequence name | Number |
|---|---|
| T3-VH | SEQ ID NO. 1 |
| T3-VL | SEQ ID NO. 2 |

**Table 2 Amino acid sequences of complementarity determining regions (CDRs) of heavy-chain and light-chain variable regions of a T3-monoclonal antibody**

| CDR classification method | Sequence name | Number/sequence |
|---|---|---|
| According to the IMGT numbering system | T3-VH CDR1 | SEQ ID NO. 3 |
| | T3-VH CDR2 | SEQ ID NO. 4 |
| | T3-VH CDR3 | SEQ ID NO. 5 |
| | T3-VL CDR1 | SEQ ID NO. 6 |
| | T3-VL CDR2 | Lys-Ala-Ser |
| | T3-VL CDR3 | SEQ ID NO. 7 |
| According to the Kabat numbering system | T3-VH CDR1 | SEQ ID NO. 8 |
| | T3-VH CDR2 | SEQ ID NO. 9 |
| | T3-VH CDR3 | SEQ ID NO. 10 |
| | T3-VL CDR1 | SEQ ID NO. 11 |
| | T3-VL CDR2 | SEQ ID NO. 12 |
| | T3-VL CDR3 | SEQ ID NO. 13 |
| According to the Chothia numbering system | T3-VH CDR1 | SEQ ID NO. 14 |
| | T3-VH CDR2 | SEQ ID NO. 15 |
| | T3-VH CDR3 | SEQ ID NO. 10 |
| | T3-VL CDR1 | SEQ ID NO. 11 |
| | T3-VL CDR2 | SEQ ID NO. 12 |
| | T3-VL CDR3 | SEQ ID NO. 13 |

**Table 3 Amino acid sequences of constant regions of a T3-monoclonal antibody**

| Sequence name | Number |
|---|---|
| T3-CH1 | SEQ ID NO. 16 |
| T3-CH2 | SEQ ID NO. 17 |
| T3-CH3 | SEQ ID NO. 18 |
| T3-CL | SEQ ID NO. 19 |

**Table 4 Nucleotide sequences of heavy-chain and light-chain variable regions of a T3 clone**

| Sequence name | Number |
|---|---|
| T3-VH | SEQ ID NO. 20 |
| T3-VL | SEQ ID NO. 21 |

**Table 5 Nucleotide sequences of heavy-chain and light-chain expression vectors**

| Sequence name | Number |
|---|---|
| Full-length sequence of the heavy-chain expression vector PUT-VH | SEQ ID NO. 22 |
| Full-length sequence of the light-chain expression vector PUT-VL | SEQ ID NO. 23 |

**Table 6 Amino acid sequences for anti-Trop2 CAR**

| Sequence name | Number |
|---|---|
| CD8 leader | SEQ ID NO. 24 |
| T3 anti-Trop2 scFv | SEQ ID NO. 25 |
| CD8 Hinge | SEQ ID NO. 26 |
| CD8 TM transmembrane domain | SEQ ID NO. 27 |
| 41BB costimulatory domain | SEQ ID NO. 28 |
| CD3ζ | SEQ ID NO. 29 |

**Table 7 Nucleotide sequences for anti-Trop2 CAR**

| Sequence name | Number |
|---|---|
| CD8 leader | SEQ ID NO. 30 |
| T3 anti-Trop2 scFv | SEQ ID NO. 31 |
| CD8 Hinge | SEQ ID NO. 32 |
| CD8 TM transmembrane domain | SEQ ID NO. 33 |
| 41BB costimulatory domain | SEQ ID NO. 34 |
| CD3ζ | SEQ ID NO. 35 |

### Example 1 Screening of anti-Trop2 phage antibodies

Genes encoding heavy-chain variable regions (VHs) and light-chain variable regions (VLs) of antibodies were amplified from healthy human B lymphocytes by PCR, and scFv fragments were expressed on the surface of phages to construct a phage antibody library. The fully human scFv phage antibody library of the present disclosure was obtained from Shanghai Unicar-Therapy Bio-Medicine Technology Co., Ltd. Anti-Trop2 antibodies were screened from the fully human scFv antibody library using phage display technology. A His tag-containing extracellular domain protein of Trop2, namely, Human TROP-2/TACSTD2 Protein, His Tag from ACRO (catalog No. TR2-H5223), was used for the screening of anti-Trop2 specific antibodies. *Escherichia coli* (*E. coli*) Tg1 was purchased from Lucigen (catalog No. 60502-2). Trypsin (catalog No. and specification: T1426-250 mg) was purchased from Sigma-Aldrich. M13KO7 helper phage was purchased from NEB (catalog No. N0315S). Mouse anti-M13 antibody (horseradish peroxidase (HRP)) was purchased from NBbiolab, catalog No. S004H-250 uL. 3,3',5,5'-tetramethylbenzidine (TMB) was purchased from eBioscience. 96-well ELISA plates were purchased from Costar (catalog No. 3590).

Formulation of 2×YT medium: 16 g/L of tryptone, 10 g/L of yeast extract, and 5 g/L of sodium chloride. Add double-distilled water to a beaker, weigh the above-mentioned tryptone, yeast extract, and sodium chloride, add them to the beaker, and stir until completely dissolved and clear. Put the weighed agar powder into a conical flask, pour in the culture medium, seal with a sealing film and then sterilize with an autoclave at 121 °C for 20 min. After cooling to 55 °C or lower, add corresponding antibiotics.

Formulation of 2×YTA agar culture plate: 10 g/L of tryptone, 5 g/L of yeast extract, 8 g/L of sodium chloride, and 15 g/L of agar powder. Add double-distilled water to a beaker, weigh the above-mentioned tryptone, yeast extract, and sodium chloride, add them to the beaker, stir until completely dissolved and clear. Put the weighed agar powder and pour in the culture medium, seal with a sealing film and then autoclave sterilize. After autoclave sterilization and cooling to approximately 55 °C (tolerable on the back of the hand), add an antibiotic to prevent inactivation of the antibiotic due to excessive temperature. Add ampicillin at a ratio of 1:1,000. For example, add 400 µL of ampicillin to 400 mL of a medium. Excessively high temperatures can lead to decreased activity or inactivation of antibiotics because most antibiotics are not heat-stable. When pouring plates, air bubbles should be avoided, and multiple plates should be stacked together to prevent excessive condensation of water droplets on dish lids caused by their low temperature. After the agar had completely solidified, the plates were inverted and allowed to stand at room temperature for 1 d to avoid premature storage at 4 °C (premature incubation at 37°C often resulted in water droplets causing bacterial colonies to merge). Resulting solid medium plates were placed in a laminar flow hood overnight and then transferred to a refrigerator. The refrigerator should be kept sterile.

Preparation of a 100 mg/mL ampicillin solution (ampicillin was purchased from Sangon Biotech (Shanghai) Co., Ltd., catalog No. A610028-0025): 100 mg of ampicillin was dissolved in 1 mL of sterile water, and a resulting solution was filtered through a 0.22 µm microporous membrane for sterilization and then stored at 4°C for later use.

Preparation of a polyethylene glycol (PEG)/NaCl solution: 20% PEG8000, 2.5M NaCl stoCL. Specific formulation: 73.1 g of NaCl, 100 g of PEG8000 (purchased from Sangon Biotech (Shanghai) Co., Ltd., catalog No. A100159-0500), and 500 mL of double-distilled water. After preparation, autoclave sterilize at 100 kPa and 121°C for 20 min, mix well.

Phage display technology refers to a biotechnology in which a DNA sequence encoding an exogenous protein or polypeptide is inserted into an appropriate position of a structural gene for a phage coat protein, such that the exogenous gene is expressed along with the phage coat protein and the exogenous protein is displayed on the surface of a phage upon phage reassembly. In the present disclosure, phage display technology was employed for solid-phase screening of anti-Trop2 antibodies.

Screening steps were as follows: Coat 10 µg/ml of Trop2-His protein (0.2 µg/µL) on a 96-well ELISA plate, seal the plate with sealing film, and incubate at 4°C overnight. Remove the antigen solution from the ELISA plate, and wash three times with phosphate-buffered saline (PBS) (aspirate with a pipette; decanting may cause contamination). Add MPBS and block at room temperature for 1 h, and then aspirate. Meanwhile, block 100 µL of the phage antibody library with 100 µL of MPBS (2% non-fat milk) at room temperature for 1 h. Then, add 200 µL of the blocked phage antibody library to a well with the antigen, and incubate at room temperature for 1 h. Remove the phage solution, wash the ELISA plate 14 times with PBST (0.1% Tween) and then wash 2 times with PBS. Add 250 µL of trypsin (100 µL of trypsin + 900 µL of 1× PBS), and incubate at room temperature for 15 min, then aspirate the eluate, transfer to a 1.5 mL EP tube, add another 250 µL of trypsin and digest for 15 minutes. During the second digestion, mix by pipetting and aspirate completely. The total volume of the digested eluate is 500 µL, transferred to an EP tube. Take 10 µL of the eluted phage solution and perform 10-fold serial dilutions with 2YT: 10^2-fold, 10^4-fold, 10^6-fold, 10^7-fold, 10^8-fold, and 10^9-fold dilutions. Take 100 µL of the 10^7, 10^8, and 10^9 diluted phage and add to 200 µL of TG1 culture at OD₆₀₀ of 0.5 to determine the titer. Additionally, take another 250 µL of the eluted phage to infect 3 mL of an TG1 *E. coli* at OD₆₀₀ of 0.5, add Helper phage, mix well, incubate in a 37°C water bath for 30 min, then serially dilute the bacterial culture at 10^2-fold, 10^4-fold, and 10^5-fold dilutions, take 200 µL of each dilution and spread onto a 75 mm 2YTA plate to determine an output titer. Centrifuge the remaining bacterial culture at 3,500 rpm for 5 min, discard part of the supernatant, then spread onto a 150 mm 2YT plate (for phage amplification). Resulting plates were labeled as output 10^2, 10^4, and 10^5, respectively, and incubated overnight in a 37 °C incubator. The remaining eluted phage solution was stored at 4°C. Scrape the plates, use 2YT medium to scrape the bacteria, take approximately 150 µL of the scraped bacteria and add to 25 mL of a 2YT Amp 2% glucose medium, shake at 220 rpm for 30 min until reaching logarithmic growth phase. Store the remaining bacterial suspension in 50% glycerol and freeze at -20°C. After shaking for 30 min to logarithmic growth phase, add 100 µL of helper phage (1 × 10¹² pfu/mL), incubate in a 37 °C water bath for 30 min after adding helper phage, and then shake at 220 rpm and 37°C for 1 h. Centrifuge at 3,500 rpm for 10 min, replace with 50 mL of 2YT medium kanamycin-ampicillin double-antibiotic medium, place in a 125 mL conical flask, and culture overnight at 30 °C and 220 rpm. Divide the 50 mL of a culture from the 125 mL conical flask into two centrifuge tubes, 25 mL each, centrifuged at 8,000 g for 10 min, retain the supernatants. Add PEG-NaCl at 1/3 or 1/4 of the supernatant volume after centrifugation to precipitate the phage, mix thoroughly, and let stand on ice for 1 h. Centrifuge at 10,000 rpm and 4 °C for 10 min, circle the white precipitate after centrifugation with a marker pen, discard the supernatant (be careful as the precipitate is easily dislodged and cannot be decanted), centrifuge again at 10,000 rpm for 1 min, resuspend the precipitate in 400 µL of 1× PBS, wash thoroughly, transfer to a 1.5 mL EP tube, centrifuge at 12,000 g for 5 min, discard the precipitate (other impurities), combine the supernatants from the two tubes and transfer to a 1.5 mL EP tube. This is the input resulting from the first round of screening. Use 200 µL for the next round of screening, and store the remaining input in a 4 °C refrigerator. Repeat the above screening steps for a total of four rounds of "adsorption-elution-amplification" for enrichment screening, then perform phage ELISA screening to identify positive monoclonal antibodies.

Phage ELISA steps: An output phage antibody library obtained from the fourth round of screening was serially diluted and used to infect 200 µL of *E. coli* TG1 at logarithmic growth phase, spread onto a 2YTA plate (ampicillin concentration: 100 mg/mL) and cultured overnight at 37°C. In two 96-deep-well plates, add 300 µL of 2YTGA (1% glucose and 100 mg/mL ampicillin) per well. Prepare 60 mL of 2YTGA. Randomly pick 92 clones from the plates of the final round of screening and add them to the deep-well plates, remaining 2 wells as TG1 controls and 2 wells as 2YTA control. Mix using a multichannel pipette, shake at 37 °C for 3.5 h to 4 h. Use a 96-well cell culture plate to preserve the bacteria. Take 100 µL of bacterial culture from the 96-deep-well plate and add 100 µL of 50% glycerol, and stor at -20°C. Take 50 µL of Helper phage and add to3.3 mL of 2YT antibiotic-free medium, mix well, then add 30 µL of the helper phage-containing medium to each well of a deep-well plate, pipette below the liquid surface, mix after addition. Incubate a 37 °C incubator for 30 min. Then shake at 220 rpm and 37 °C for 1 h. Add 400 µL of a 2YT AMP+KANA+medium (ampicillin and kanamycin double antibiotic) to each well of the deep-well plate, making a final volume of 600 µL per well, culture overnight at 220 rpm and 30°C. Antigen coating: Coat one 96-well ELISA plate with Trop2-His antigen and another 96-well ELISA plate with bovine serum albumin (BSA) (to detect non-specific binding), both at a concentration of 1 µg/mL, 4 °C overnight. ELISA identification: The next day, take out the 96-deep-well plate containing the cultured phage, let it stand to allow bacterial cells to precipitate, and collect the supernatant. Take the 96-well ELISA plate coated with antigen overnight at 4°C, block each well with 200 µL of 2% non-fat milk for 1 h, and then wash three times with PBST. During the milk blocking of the ELISA plate, take 120 µL of Phage supernatant and add 120 µL of 2% non-fat milk, block the phage supernatant using a dilution plate. After blocking, add the corresponding supernatant as the primary antibody. The supernatants added to the deep-well plate and the 96-well ELISA plate correspond one-to-one, 100 µL/well, incubate for 1.5 h and wash 6 times with PBST. Add anti-M13-HRP (anti-M13 phage) diluted 1:5000 in 2% non-fat milk, 100 µL/well, and incubate for 1 h. Add TMB, 100 µL/well, develop color for 10 min. Add 2 M H₂SO₄, 100 µL/well, to stop the color development. Read the OD₄₅₀ using a microplate reader. The negative standard is OD₄₅₀ < 0.2. Pick the positive clones identified by Phage ELISA results and send to Sangon Biotech (Shanghai) Co., Ltd. for sequencing to determine the diversity of the positive clone sequences.

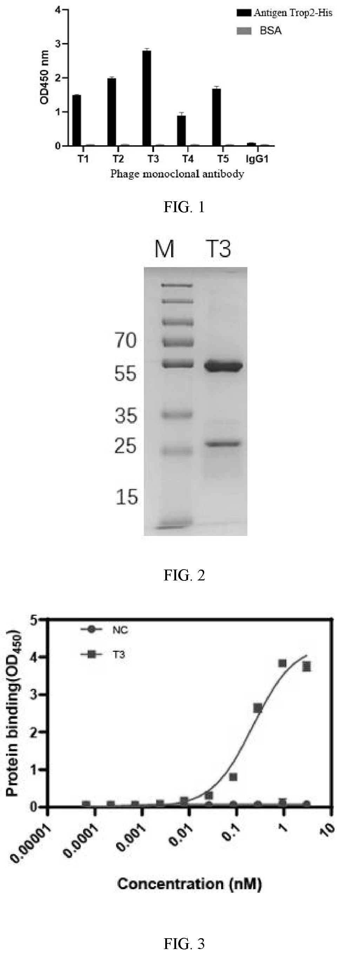

By screening anti-Trop2 phage antibodies using ELISA, the following 5 positive clones were identified (FIG. 1): anti-Trop2 positive clone 1 (T1), anti-Trop2 positive clone 2 (T2), anti-Trop2 positive clone 3 (T3), anti-Trop2 positive clone 4 (T4), and anti-Trop2 positive clone 5 (T5). The five positive clones all showed specific binding to the Trop2 antigen, among which T3 exhibited relatively high binding activity. Amino acid sequences of a heavy-chain variable region VH and a light-chain variable region VL of the T3 monoclonal antibody are shown in Table 1 (T3-VH: SEQ ID NO. 1 and T3-VL: SEQ ID NO. 2). Nucleotide sequences for the heavy-chain variable region VH and the light-chain variable region VL of the T3 monoclonal antibody are shown in Table 4 (T3-VH: SEQ ID NO. 20 and T3-VL: SEQ ID NO. 21). In the present disclosure, the T3 monoclonal antibody with relatively high binding activity was selected for subsequent experiments.

### Example 2 Identification of anti-Trop2 antibodies

A source of a Trop2 antigen is the same as that in Example 1. A plasmid DNA miniprep kit was purchased from Corning*. E. coli* Top10 competent cells were purchased from TIANGEN Biotech Co., Ltd. An HRP-conjugated anti-Fc antibody was purchased from Bethyl. TMB was purchased from eBioscience. An SDS-PAGE gel rapid preparation kit was purchased from Beyotime. A protein A pre-packed column for protein purification was purchased from Yeasen.

DMEM was purchased from Gibco. Mouse anti-human IgG-Fc (HRP) was purchased from Bethyl. A PE anti-human Fc antibody was purchased from Biolegend. A BxPC-3 pancreatic cancer cell line, an MDA-MB-231 triple-negative breast cancer cell line, and an H1975 NSCLC cell line were purchased from the Chinese Academy of Sciences, Shanghai, China. A heavy-chain expression vector PUT-VH and a light-chain expression vector PUT-VL were purchased from Shanghai Unicar-Therapy Bio-Medicine Technology Co., Ltd., and corresponding nucleotide sequences are shown in Table 5 (the full sequence of the heavy-chain expression vector PUT-VH is shown in SEQ ID NO. 22 and the full sequence of the light-chain expression vector PUT-VL is shown in SEQ ID NO. 23).

The heavy-chain variable region VH of the scFv of the positive T3 monoclonal antibody was constructed into the heavy-chain expression vector PUT-VH (its nucleotide sequence is shown in Table 5) to produce a T3 VH plasmid. The light-chain variable region VL of the scFv of the positive T3 monoclonal antibody was constructed into the light-chain expression vector PUT-VL (its nucleotide sequence is shown in Table 5) to produce a T3 VL plasmid. The T3 VH plasmid and the T3 VL plasmid were transformed into the *E. coli* Top10 through calcium chloride transformation, respectively. Single colonies were picked, expanded in culture, and then plasmid were extracted. The extracted plasmids (the T3 VH plasmid and the T3 VL plasmid were at a ratio of 1:1) were co-transfected into 293T cells through calcium chloride transfection. A resulting supernatant was collected and purified using a Protein A affinity column. The identification of a monoclonal antibody mainly involved determining the molecular weight of the monoclonal antibody and the binding activities of the monoclonal antibody to the antigen at both the protein and cellular level. SDS-PAGE was used to determine whether the molecular weight of the monoclonal antibody is correct, and ELISA and flow cytometry were used to detect the antibody-antigen binding.

SDS-PAGE gel electrophoresis identification steps: 1) Gel preparation: a) Plate washing: wipe two glass plates clean, then wipe the plates dry with alcohol. b) Assembly of the two glass plates: align the two glass plates neatly, clamp both sides with clamps, and fix the two glass plates onto the base. Add deionized water to check for leakage. Insert the matching comb and draw a line below comb to indicate the gel pouring position (stacking gel). c) Preparation of a 1.5 mm-thick gel using a PAGE gel rapid preparation kit: 1. Take equal volumes of a resolving gel solution and resolving gel buffer (4 mL each) and thoroughly mix. 2. Add 80 µL of the improved accelerator (ammonium sulfate) to the mixed solution obtained in the step 1, and mix. 3. Pour all the mixed solution obtained in the step 2 into the gel casting glass plates, not too far from the marked line, leaving space for the stacking gel. 5 min later, add approximately 2 mL of water to flatten the resolving gel. The appearance of a refractive line between the water and the gel indicates solidification. 4. Pour off the water in an upper water layer and absorb residual water with a filter paper. 5. Take equal volumes of the stacking gel solution and a colored stacking gel buffer (1 mL each), and thoroughly mix. Shake well before use. 6. Add 20 µL of the improved accelerator to the mixed solution obtained in the step 5, and mix. 7. Pour the mixed solution obtained in the step 6 into the gel casting glass plates. 8. After the stacking gel has solidified (10 min to 15 min), remove the comb, and the gel is ready for electrophoresis. 9. Sample loading and electrophoresis: Mix 10 µL of protein with 10 µL of 2× loading buffer at a ratio of 1:1, heat in a 100°C boiling water bath for 10 min, centrifuge at 12,000 for 1 min. Load a total of 20 µL of a sample onto the SDS-PAGE gel. Run at 90 V in the stacking gel and at 120 V in the resolving. The electrophoresis time is approximately 2 h. 2) 30 min of Coomassie Brilliant Blue staining and destaining: a. After the electrophoresis, place the gel into an appropriate amount of Coomassie Brilliant Blue staining solution, ensuring the solution fully covers the gel. b. Place on a horizontal shaker or an orbital shaker, and shake slowly. Stain at room temperature for 1 hour. c. Pour out the staining solution. The staining solution could be recovered and reused at least 2 to 3 times. d. Add an appropriate amount of destaining solution, ensuring it fully covers the gel. The destaining solution formula is: 40% of ethanol, 10% of acetic acid, and 50% of distilled water. e. Place on a horizontal shaker or an orbital shaker and shake slowly. Destain at room temperature for 4-24 hours, changing the destaining solution 2 to 4 times during this period, until the blue background is almost completely removed and the protein band staining effect meets expectations. Protein bands appear after 1-2 hours of destaining. f. After destaining is complete, store the gel in water for subsequent photography.

ELISA steps for determining the affinity of the antibody for the Trop2 antigen: 1) Coat the Trop2-His antigen onto a 96-well ELISA plate at a concentration of 1 µg/mL, seal the plate with a sealing film, and incubate at 4°C overnight. 2) After overnight coating of the ELISA plate, wash once with 0.05% PBST, and then block with non-fat milk powder for 1 h, and dilute the primary antibody during this period. 3) Serially dilute the primary antibody 3.3-fold using 2% non-fat milk with a starting concentration of 5 µg/mL to produce a total of 12 primary antibody concentrations. 4) After blocking of the ELISA plate is complete, add the primary antibody at 100 µL/well. After adding the primary antibody, incubate for 1 h. 5) Wash 6 times with 0.05% PBST. 6) Add secondary antibody, mouse anti-human IgG-Fc (HRP), diluted at 1:1,000 with 2% non-fat milk, 100 µL/well, incubate for 1 h. 7) Wash 6 times with 0.05% PBST. 8) Add 100 µL/well TMB for color development for 10 min. Stop the reaction\ if a color was too deep. 9) Add 100 µL of sulfuric acid (2M) per well to stop the color development reaction. 10) Measure the absorbance with a microplate reader (450 nm).

Flow cytometry detection of the binding of the obtained T3 anti-Trop2 monoclonal antibody to cell surface Trop2, detection steps: 1. Dilute the antibody to a final concentration of 5 µg/mLwith pre-chilled 1× PBS, final volume of 200 µL. 2. Digest the cells to be tested, adjust the density of the cells to be tested to 1 × 10⁶ cells/mL with culture medium, add 1 mL of the cell suspension to each tube (depending on the amount of cells), centrifuge at 4°C, and discard the supernatant. 3. Wash once with 1 mL of pre-chilled 1× PBS at 1,500 rpm for 5 min. 4. Add 200 µL of the diluted primary antibody (T3 monoclonal antibody) to resuspend the cells, and place on ice for 60 min. 5. Centrifuge and discard the supernatant, wash once with 1 mL of pre-chilled 1× PBS at 1,200 rpm for 5 min. 6. Dilute the secondary antibody with pre-chilled 1× PBS containing 5% of BSA, add 1 µL of secondary antibody anti-Fc (PE) per tube to resuspend the cells, and place on ice for 30 min. 8. Wash once with 1 mL of pre-chilled 1× PBS at 1,500 rpm for 5 min. 9. Resuspend the cells in 200 µL of pre-chilled 1× PBS and analyze on a flow cytometer.

The supernatant purified by AKTA using a Protein A column was tested by SDS-PAGE electrophoresis. The SDS-PAGE electrophoresis results (FIG. 2) showed that the molecular weights of the heavy chain and light chain of the T3 monoclonal antibody in the supernatant were consistent with expectations (FIG. 2). After serial dilution of the T3 monoclonal antibody, ELISA was used to detect the binding of the antibody to the Trop2 antigen. The results shown in FIG. 3, indicated that the screened antibody bound to the Trop2 antigen with high specificity and activity (FIG. 3). NC is the negative antibody control, which is an anti-human CD7 antibody from Shanghai Unicar-Therapy Bio-Medicine Technology Co., Ltd., antibody No. PUT644, obtainable by the method described in the patent CN113603778A.

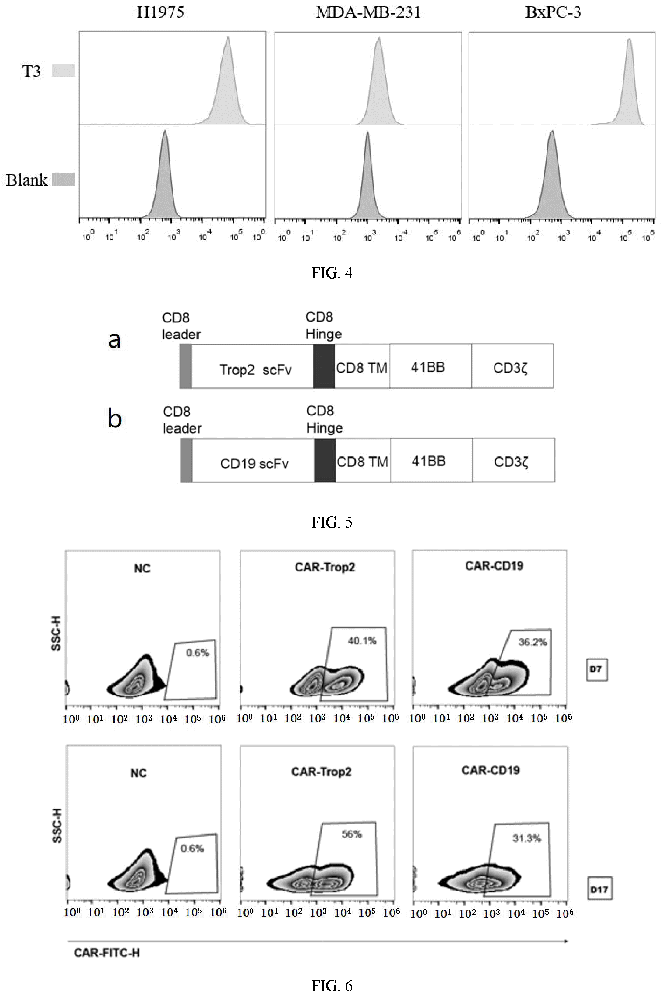

Further identification of the antibody's binding to cell surface Trop2 antigen by flow cytometry. In FIG. 4, the Blank group represents cells without antibody added, and the T3 group shows the binding of the T3 monoclonal antibody to the cells. It was shown that the anti-Trop2 antibody bound strongly to three solid tumor cell lines: triple-negative breast cancer MDA-MB-231, NSCLC H1975, and pancreatic cancer Bxpc3. The binding strength can be characterized by the median fluorescence intensity (MFI). According to FlowJo analysis results, the MFI for each group was as follows: MFI for the binding of H1975 to T3 was 65,580 (621). MFI for the binding of MDA-MB-231 to T3 was 3,150 (938). MFI for the binding of Bxpc3 to T3 was 174,408 (557). Each value in parentheses indicates the corresponding MFI for the Blank control cells.

The pancreatic cancer Bxpc3, the triple-negative breast cancer MDA-MB-231, and the NSCLC H1975 all showed strong binding (FIG. 4), indicating that the anti-Trop2 antibody screened in the present disclosure could bind to the Trop2 antigen expressed on the cell surface with high binding strength.

More and more researchers are focusing on antibody production. Currently, hybridoma antibody technology has been relatively well-established, and many murine monoclonal antibody drugs have already been used in clinical practice. However, murine monoclonal antibody drugs have immunogenicity, which greatly limits their development prospects. The antibody screened in the present disclosure is a fully human antibody screened using phage display technology. Compared with traditional antibodies produced by immunizing animals, the fully human antibody has low immunogenicity and represents the main direction of future development.

scFv consists of a heavy-chain variable region (VH) and a light-chain variable region (VL) linked together by a peptide linker. The generation and affinity of tumor-specific scFv are fundamental to the safety and efficacy of CAR-T therapy. Currently, there are two major technologies for constructing scFv: hybridoma technology and phage display technology, corresponding to antibody types ranging from 100% murine antibodies to 100% human antibodies. A murine antibody refers to a monoclonal antibody whose constant region and variable region sequences are both derived from mouse genes, with 100% of murine origin. The preparation of murine monoclonal antibodies relies on hybridoma technology. Specifically, this involves exposing mice to a specific antigen, then screening and extracting the B lymphocytes that produce antibodies from them; these cells are then fused with mouse myeloma cells that cannot produce antibodies. Subsequently, mouse hybridoma cells that do not undergo apoptosis, can proliferate indefinitely, and are capable of secreting a specific antibody are screened. Murine antibodies primarily have two advantages: (1) As first-generation monoclonal antibodies, murine antibodies have relatively well-established and thorough basic research and preparation techniques, with relatively low costs. (2) The maturity of hybridoma technology ensures consistent quality across different batches of murine antibodies and enables easy replication. However, murine antibodies also have significant limitations. On the one hand, murine monoclonal antibodies have strong immunogenicity and are prone to inducing a human anti-mouse antibody (HAMA) response (that is, a murine antibody itself is recognized as an antigen by the human immune system, and thus can trigger an unnecessary immune response). In mild cases, this accelerates antibody clearance from the human body and shortens the half-life, thereby affecting therapeutic efficacy in severe cases, it can cause serious immune disorders. On the other hand, the constant region of a murine antibody has a different sequence from the human constant region, resulting in weak subsequent immune responses. Due to the above two limitations, murine antibodies have largely eliminated from clinical applications. However, due to low costs, murine antibodies are still widely used in laboratory environments. This has also contributed to the emergence of subsequent second-generation and third-generation human monoclonal antibodies.

Human antibodies can be further subdivided into the following three major categories based on the proportion and location of a human-derived component. A Human-mouse chimeric monoclonal antibody has a constant region derived from a human and a variable region derived from a mouse (about 30% of a murine component). A human monoclonal antibody has a constant region derived from a human and a variable region that is a combination of human and murine sequences (about 10% of a murine component). A fully human monoclonal antibody has both constant and variable regions derived from a human (0% of a murine component). Human antibodies are generally superior to murine antibodies. This is mainly reflected in the following aspects: 1) Fully human antibodies can effectively weaken the human anti-mouse antibody response, and can reduce toxic and side effects while lowering immunogenicity. 2) they are less likely to be recognized by the immune system, which significantly prolongs the retention time of fully human antibodies *in vivo*.

The scFv selected by the phage display technology in the present disclosure is a 100% human antibody with 0% of a murine component. Characteristics, advantages, and disadvantages of human and murine antibodies are further amplified in CAR-T therapy. Advantages of human antibodies in CAR-T immunotherapy are mainly reflected in the following three aspects:
1. The efficacy of human antibodies is more robust: Currently, extracellular scFv fragments for constructing CARs in clinical trials are mostly murine monoclonal antibodies. However, a human leukocyte antigen (HLA) T cell-mediated immune response triggered by the specific binding of murine scFv in CAR to an immunogenic epitope of a target antigen, on one hand, hinders the interaction between the CAR and the target antigen to inhibit the function of CAR-T cells, and on the other hand, affects the activation and proliferation of cells, and even leads to the inactivation of CAR-T cells in a patient. In contrast, human scFv enables high efficacy stability by reducing the immunogenicity of CAR.
2. Human antibodies can effectively prolong the time to relapse: After CAR-T therapy, factors such as CAR-T cell exhaustion and changes in a tumor microenvironment may lead to disease relapse. HAMA response triggered by the high immunogenicity of a murine antibody shortens a half-life of the murine antibody in the human body and thus may shorten the time to disease relapse.
3. The efficacy of secondary treatment with murine antibodies is reduced: For tumors targeting CD19, after the first application of murine CAR-T therapy and the disease relapse, the second application of the murine CAR-T therapy leads to reduced efficacy. The high immunogenicity of murine antibodies may induce the production of anti-murine antigen antibodies in the body, manifesting as drug resistance. In such cases, the second treatment with human or fully human CAR-T can achieve effective remission.

The following examples will demonstrate, from the aspects of CAR and CAR-T, the preparation process of a CAR (anti-Trop2 CAR) are prepared using the anti-Trop2 antibody screened in the present disclosure and T cells modified with this CAR (anti-Trop2 CAR-T), their LDH -based cytotoxicity on different target cells and cytokine release effects, as well as the specific killing effects of anti-Trop2 CAR-T on xenograft tumors of NSCLC and triple-negative breast cancer cells through *in vivo* animal experiments. However, this does not mean that the anti-Trop2 antibody screened in the present disclosure can only be used for preparing CAR and T cells modified with the CAR. The anti-Trop2 antibody itself as a Trop2-binding antibody or Trop2 antigen-binding fragment, the nucleic acid encoding it, the vector containing the nucleic acid, and the host cell containing the nucleic acid or the vector all possess considerable biological values. Due to limited space, research data on CAR and CAR-T are highlighted here as examples.

### Example 3 Construction of anti-Trop2 CAR and packaging of lentivirus

A lentiviral backbone plasmid vector was PSB1819 obtained from Shanghai Unicar-Therapy Bio-Medicine Technology Co., Ltd. pPac-R, pPac-GP, and pEnv-G were also obtained from Shanghai Unicar-Therapy Bio-Medicine Technology Co., Ltd. DMEM was obtained from Gibco. TOP10 competent cells were obtained from TIANGEN Biotech Co., Ltd.

scFv of the anti-Trop2 positive clone 3 obtained in Example 1 was seamlessly cloned into the lentiviral backbone plasmid vector PSB1819 (from Shanghai Unicar-Therapy Bio-Medicine Technology Co., Ltd.) to produce a target plasmid. This was transformed into *E. coli* TOP10, identified by sequencing, expanded in culture, and performed plasmid midi-prep. After plasmid extraction, 293T cells were then transfected with the target plasmid and lentiviral packaging plasmids pPac-R (from Shanghai Unicar-Therapy Bio-Medicine Technology Co., Ltd.), pPac-GP (from Shanghai Unicar-Therapy Bio-Medicine Technology Co., Ltd.), and pEnv-G (from Shanghai Unicar-Therapy Bio-Medicine Technology Co., Ltd.).

Plate the 293T cells to be transfected in 10 cm culture dishes. When the cell density reached approximately 70%, proceed with lentiviral packaging as follows: Take out the 293T cells and observe their condition. For a total of 10 dishes of 293T cells, if the cells are in good condition with 80%-90% confluency, discard the culture medium and carefully add 9 mL of a 4% DMEM complete medium in a 15 mL centrifuge tube, add 2,500 µL of CaCl₂, three packaging plasmids (80 µg of pPac-R, 100 g of pPac-GP, and 60 g of pEnv-G), and 220 µg of the target plasmid anti-Trop2 CAR. Immediately add bacterial endotoxin test water to a total volume 5,000 µL. Add 5,000 µL of Hepes buffered saline (HBS), and after addition, continuously shake for about 20 s. Take out the 293T cells from the incubator, add 1 mL of the mixed transfection reagent to each dish, gently shake to mix evenly, and then place back in the incubator. Discard the culture supernatant 6 h after transfection, and add 10 mL of 4% DMEM complete medium. Continue to add another 10 mL of 4% DMEM complete medium24 h after transfection. Collect the viral supernatant at 24 h, 48 h, and 72 h of culture and store at 4 °C. After all viral supernatants are collected, filter the viral supernatant using a 0.45 µm filter to remove cell debris. Then add PEG 8000 to concentrate the virus. Incubate at 4°C for 12 h, then centrifuge at 3,000 rpm for 15 min. After centrifugation, discard the supernatant, and add an appropriate amount of sterile PBS to dissolve the viral precipitate. Aliquot the dissolved virus and store in a -80 °C refrigerator to obtain the recombinant lentiviral vector. The recombinant lentiviral vector contains the nucleic acid fragment encoding the anti-Trop2 CAR.

The construction of CARs in anti-Trop2 CAR-T cells and anti-CD19 CAR-T cells is shown in FIG. 5. A fragment encoding the anti-Trop2 CAR is a fragment encoding CD8 leader-anti-Trop2 scFv-CD8 Hinge-CD8 TM-41BB costimulatory domain-CD3ζ intracellular signaling peptide. Tables 6 and 7 show an amino acid sequence and a nucleotide sequence for each fragment. Since the anti-Trop2 scFv is derived from the T3 clone, it is denoted as T3 anti-Trop2 scFv in Tables 6 and 7.

### Example 4 Preparation and characterization of anti-Trop2 CAR-T cells

CD4/CD8 magnetic beads, an anti-CD3 antibody, and an anti-CD28 antibody were all obtained from Miltenyi Biotec. IL-2 was obtained from PeproTech. An AIM-V medium was obtained from Gibco. FITC-Protein L was obtained from Biolegend.

CD4⁺ CD8⁺ T cells were isolated from human peripheral blood mononuclear cells using CD4 and CD8 magnetic beads, then placed in T25 flasks coated with the anti-CD3 antibody and the anti-CD28 antibody, and subjected to activation for 24 h. After complete activation of T cells, the recombinant lentiviral vector prepared in Example 2 was added at multiplicity of infection (MOI) of 120. After 48 h of infection, the cells were washed twice with PBS and resuspended in fresh complete medium, and then the transfection efficiency of T cells transduced with the lentivirus was detected by flow cytometry using FITC-Protein L. Protein L is an Ig-binding protein that can specifically bind to a light chain of Ig.

The construction of CARs in anti-Trop2 CAR-T cells and anti-CD19 CAR-T cells is shown in FIG. 5. After transducing T cells with the lentivirus, the infection efficiency was detected by flow cytometry on day 7 (D7) and day 17 (D17) after incubation with Protein L-FITC, and the infection efficiency was above 40% in all cases (FIG. 6). The day of T cell activation was designated day 0 (D0), activation was carried out for 24 h followed by transduction, and the day of adding the lentivirus to transduce T cells was designated day 1 (D1).

### Example 5 Detection of LDH-based cytotoxicity and cytokine release of anti-Trop2 CAR-T cells against different target cells

An LDH-based cytotoxicity assay kit was the CytoTox 96 Non-Radioactive Cytotoxicity Assay purchased from Promega, catalog No. REF: G1782. An AIM-V medium was purchased from Gibco. A549 (Trop2-negative) was purchased from the Cell Bank of the Chinese Academy of Sciences in Shanghai, China. A CD19-overexpressing myeloid leukemia cell line K562-CD19 was purchased from Shanghai Unicar-Therapy Bio-Medicine Technology Co., Ltd.

The LDH release assay was used to detect the killing efficiency of effector cells (anti-Trop2 CAR-T cells) against target cells. LDH-based cytotoxicity assay was conducted according to instructions of the kit: 1) Collect the target cell suspension: For adherent cells, discard the culture supernatant, wash the dish bottom with 10 mL of normal saline, discard the saline, add 1 mL of trypsin to each dish to digest the cells. The digestion time depends on the characteristics of the cell line. Observe under a microscope that the cells are no longer non-adherent, are suspended, and have rounded up, then add 3 mL of complete medium to stop the digestion. Centrifuge at 1,500 rpm for 5 min, discard the supernatant, add 5 mL of PBS to resuspend, centrifuge at 1,500 rpm for 5 min, discard the supernatant. 2) Target cell plating: Resuspend the target cells in 1 mL of AIM-V cytotoxicity medium (AIM-V + 4% FBS). Count using a hemocytometer. Add 1 × 10⁴ target cells per well in a 96-well plate. Prepare the target cell suspension with the cytotoxicity medium according to the required cell amount. After preparation, plate the target cells in the 96-well cell culture plate, centrifuge at 250 g for 5 min, and place in a 37 °C CO₂ incubator for 2 h to promote target cell adherence. 3) Collect the effector cell suspension: Mix the T cells evenly and count using a hemocytometer. According to the required amount of effector cells for cytotoxicity, take the effector cells, centrifuge at 1,500 rpm for 5 min, resuspend the effector cells in 1 mL of AIM-V cytotoxicity medium (AIM-V + 4% FBS), and count using trypan blue staining. 4) Each well of the 96-well plate already contains 1 × 10⁴ target cells. Set the effector-to-target (E:T) ratio. For 2.5:1, add 2.5 × 10⁴ effector cells per well; for 5:1, add 5 × 10⁴ effector cells per well; for 10:1, 1 × 10⁵ effector cells per well. A volume added per well was 50 µL. According to the system calculation, for the E:T ratio of 10:1 condition with 1 × 10⁵ effector cells/well, prepare 1 volume of cells, then perform 2-fold serial dilutions using the cytotoxicity medium to prepare effector cell suspensions for the E:T ratios of 5:1 and 2.5:1. 5) After the target cells have adhered for 2 h and the effector cell suspensions are prepared, proceed with plating. Set up an effector cell blank group, a target cell blank group, a target cell maximum lysis group, and a medium blank group (these groups must be set for killing efficiency calculation). After plating, seal the 96-well plate with a sealing film, place in a centrifuge, centrifuge at 250 g, acceleration 3 and deceleration 1 for 5 min, remove the sealing film and incubate in a 37 °C constant temperature incubator for 24 h. 6) 45 min before LDH-based cytotoxicity assay, add 10 µL of lysis solution to each of the target cell maximum lysis wells, and continue incubation in the 37 °C constant temperature incubator. 7) Take the Substrate out from -20 °C, allow it to reach room temperature, and gently invert and shake to dissolve the Substrate. 8) Take out the 96-well plate, seal it with sealing film, place in a centrifuge, and centrifuge at 250 g, acceleration 3 and deceleration 1 for 5 min. Transfer 50 µL of supernatant from each well to a new 96-well plate, then add 50 µL of the substrate to each well, incubate in the dark for 10 min to 15 min. Measure the absorbance at 490 nm using a microplate reader. 9) Killing efficiency calculation: cytotoxicity (%) = experimental group killing/maximum killing × 100% = (experimental well - effector cell blank well - target cell blank well + culture medium background well)/(target cell maximum lysis well - culture medium volume correction well - target cell well + culture medium background well) × 100%.

Cytokine detection steps: After co-incubation of effector T cells with target cells, collect the supernatant into 1.5 mL EP tube for cytokine detection. a. Sample treatment: Centrifuge the cell suspension at 1,500 rpm for 3 min, then collect the supernatant into a 1.5 mL EP tube. b. Standard preparation: Add 2 mL of standard diluent to dilute the standard lyophilized powder, let stand at room temperature for 15 min to produce a standard solution. Then, perform 2-fold serial dilutions of the standard (10 gradients from a maximum concentration to the blank diluent). c. Add 50 µL of standard or sample to a new EP tube, then add 50 µL of magnetic beads and 50 µL of detection antibody, mix well using a vortex mixer, and incubate at room temperature for 3 h. d. Add Wash Buffer, wash twice with, discard the supernatant, add 200 µL of wash buffer to resuspend, and analyze on a flow cytometer for cytokines IL-2 / IL-4 / IL-6 / IL-10 / IFN-γ / TNF.

LDH-based cytotoxicity assay results showed that anti-CD19 CAR-T cells could only kill CD19-expressing K562 cells, but could not kill the other five cell lines. Compared with the control anti-CD19 CAR-T cells, anti-Trop2 CAR-T cells exhibited high specific cytotoxicity against four solid tumor cell lines with high Trop2 expression levels: a pancreatic cancer cell line Bxpc3, a bladder cancer cell line T24, and NSCLC cell lines PC-9 and H1975, with remarkable dose dependence. The higher the number of CAR-T cells, the higher the killing level. In contrast, anti-Trop2 CAR-T cells exhibited no cytotoxicity against Trop2-negative cell lines, (Trop2-negative NSCLC cell line A549 and CD19-overexpressing myeloid leukemia cell line K562-CD19) (FIG. 7).

In addition, cytokines released by anti-Trop2 CAR-T cells after being co-incubated with different solid tumor cell lines *in vitro* were detected. The results show:
Compared with the control group of anti-CD19 CAR-T cells, anti-Trop2 CAR-T cells co-incubated with high Trop2 expression levels solid tumor cell lines (the pancreatic cancer cell line Bxpc3 and the triple-negative breast cancer cell line MDA-MB-231) for 24 h released large amounts of IFN-γ, TNF-α, and IL-2. In contrast, cytokine levels with the Trop2-negative cell line A549 were relatively low (FIG. 8).

The above *in vitro* results proved that anti-Trop2 CAR-T cells can be stimulated and activated by Trop2-positive target cells *in vitro,* produce a series of cytokines associated with immune activation, undergo specific proliferation, and effectively kill target cells. Consequently, to investigate whether anti-Trop2 CAR-T cells can exert a similar anti-tumor effect *in vivo*, experiments in mice were conducted for validation. A human pancreatic cancer mouse model was constructed to investigate the anti-tumor efficacy of anti-Trop2 CAR-T cells *in vivo*.

### Example 6 Experiments of anti-Trop2 CAR-T cells in animals

Preparation of tumorigenic cells: MDA-MB-231 (triple-negative breast cancer cell line) and H1975 (NSCLC cell line) were obtained from the Cell Bank of the Chinese Academy of Sciences in Shanghai, China.

Animal preparation: Female NCG mice (NOD/ShiLtJGpt-Prkdc em26Il2rg em26/Gpt, GemPharmatech Co., Ltd., Jiangsu, strain type: KnoCL out, strain number: T001475, background: NOD/ShiltJGpt), aged 6 weeks and weighing 20 g to 25 g, were ordered one week before inoculation. Animal feeding and experimental operations were carried out strictly in accordance with specific-pathogen-free (SPF)-grade standards. The animal experiments were approved by the Ethics Committee of East China Normal University and strictly followed the guidelines of the Animal Research Committee. The mice were housed in a SPF environment at a room temperature of 26 °C, fed with sterile feed and sterile water. After acclimating to the environment for 7 d, the mice were subjected to the relevant experiments.

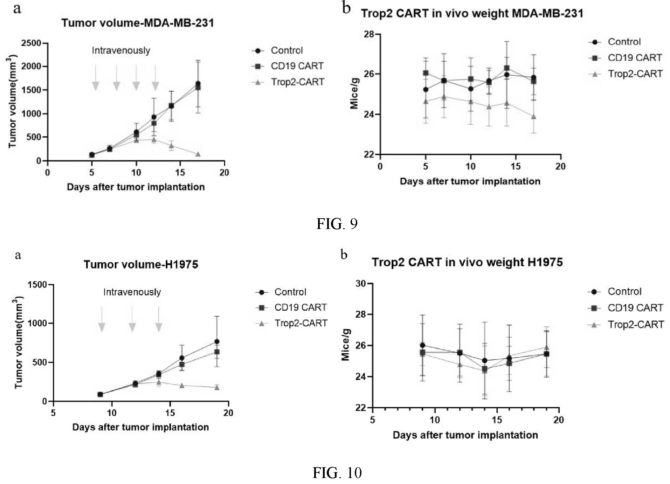

Subcutaneous xenograft modeling in NCG mice and *in vivo* experimental steps: 1) Process and collect adherent target cells: Take Bxpc3 cells at 80% to 90% confluency, add 3 mL of trypsin per T75 flask, digest for 6 min to 8 min, add 6 mL of RPMI 1640 complete medium per flask to terminate the digestion, centrifuge at 1,500 rpm, acceleration 9 and deceleration 9 for 5 minutes, discard the supernatant, then resuspend the pellet in RPMI 1640 basal , mix well, centrifuge at 1,500 rpm, acceleration 9 and deceleration 9 for 5 minutes, discard the supernatant, repeat the wash once. 2) Inoculation: After washing twice, count using trypan blue staining, determine cell viability (95% or more), adjust the cell density to 5.0 × 10⁷/mL with basal medium. Disinfect the skin under the forelimb axilla of an NCG mice with 75% alcohol. After mixing the cell suspension evenly, inject 100 µL of the cell suspension subcutaneously using an insulin syringe, then, slowly withdrawn the needle, and continue SPF rearing. 3) Mouse grouping: When a tumor volume reached 100 mm³, the mice were randomly grouped. Change the bedding regularly, observe the general conditions and feeding / activity abilities of the mice, measure the body weight and tumor volume of the mice once every two days. 4) Preparation of CAR-T cells: Use AIMV complete medium (5% of FBS + 1,000 IU/mL of IL-2), culture in a 37 °C, 5% CO₂ incubator. Observe cell growth status every two days, take microscope photos, count, replenish medium. Expand T cells *in vitro* until the required cell quantity for infusion. Before infusion, test for sterility, mycoplasma, and CAR transduction efficiency. Treatment of CAR-T cells: Take the CAR-T cells, centrifuge at 1,500 rpm, acceleration 9 and deceleration 9 for 5 min, discard the supernatant, wash twice with AIMV basal medium, then count using trypan blue staining, determine cell viability, then adjust the T cell density to 1.0 × 10⁸/mL with basal medium for infusion. 5) Infusion of CAR-T cells: When the tumor volume reached 100 mm³ in mice, the infusion of CAR-T cells was initiated. The CAR-T cell infusion schedule is: 1 × 10⁷ cells/time, for a total of 4 infusions, every 4 days (FIG. 10). Infusion procedure: Disinfect the tail of the NCG mouse with 75% alcohol, mix the cell suspension evenly, inject 100 µL of the CAR-T cell suspension into the tail vein of the mouse using an insulin syringe, then slowly withdrawn the needle, disinfect the wound and apply pressure to stop bleeding for 20 s, then continue SPF rearing.

A specific *in vivo* experimental method for anti-Trop2 CAR-T cells in an animal model of a triple-negative breast cancer cell line MDA-MB-231 was as follows: Inject 5 × 10⁶ MDA-MB-231 target cells into 6-week-old NCG mice via a tail vein. 5 days after target cell inoculation, the tumor volume was approximately 100 mm³. Tumor-bearing mice were randomly divided into three groups, with 6 mice in each group. The experimental mice received 4 times CAR-T cell infusions (1 × 10⁷ cells/mouse/time), with a 3-day interval between infusions.

A specific *in vivo* experimental method for anti-Trop2 CAR-T cells in an animal model of a lung cancer cell line H1975 was as follows: Inject 5 × 10⁶ H1975 target cells into 6 to 8-week-old NCG mice via a tail vein. 9 days after the target cell inoculation, the tumor volume reached approximately 100 mm³. Tumor-bearing mice were randomly divided into three groups, with 5 mice in each group. The experimental mice received 4 times CAR-T cell infusions (1 × 10⁷ cells/mouse/time), with a 3-dayinterval between infusions.

During the treatment process, a tumor size, a body weight, and an activity status of each mouse were continuously observed and recorded. Tumor size data was statistically analyzed. The experimental results showed that the anti-Trop2 CAR-T cell group could significantly inhibit tumor growth compared with the anti-CD19 CAR-T cell group and the untreated group. FIG. 9 shows the specific killing of MDA-MB-231 triple-negative breast cancer cells *in vivo* and significant inhibition of tumor growth by anti-Trop2 CAR-T cells, wherein a shows tumor volume curves and b shows mouse body weight change curves. Compared with mice in the control group and the anti-CD19 CAR-T treatment group, mice receiving anti-Trop2 CAR-T therapy showed significant tumor volume reduction, indicating that anti-Trop2 CAR-T cells could significantly inhibit the growth of MDA-MB-231 triple-negative breast cancer xenograft tumors. FIG. 10 shows the specific killing of H1975 lung cancer cells *in vivo* and significant inhibition of tumor growth by anti-Trop2 CAR-T cells, wherein a shows tumor volume curves and b shows mouse body weight change curves. Compared with mice in the control group and the anti-CD19 CAR-T treatment group, mice receiving anti-Trop2 CAR-T therapy showed significant tumor volume reduction, indicating that anti-Trop2 CAR-T cells could significantly inhibit the growth of H1975 lung cancer xenograft tumors. In addition, mice receiving anti-Trop2 CAR-T therapy showed no significant body weight loss, indicating that anti-Trop2 CAR-T cells were well tolerated in mice.

Significant anti-tumor effects were achieved in mouse models of both the lung cancer cell line H1975 and the triple-negative breast cancer cell line MDA-MB-231 (FIG. 9 and FIG. 10).

The in vivo results in the NCG mouse models showed that anti-Trop2 CAR-T cells could specifically kill xenograft tumors of NSCLC and triple-negative breast cancer cells *in vivo*.

All publications and patent applications cited in this specification are incorporated herein by reference as if each individual publication or patent application were specifically and individually indicated to be incorporated herein by reference. Further, any theories, mechanisms, proofs, or findings described herein are intended solely to enhance the understanding of the present disclosure and are not intended to limit the present disclosure in any way. Although the present disclosure has been illustrated and described in detail in the accompanying drawings and the above description, the present disclosure should be regarded as illustrative rather than restrictive.

## Claims

1. A trophoblast cell surface antigen 2 (Trop2)-binding antibody or Trop2 antigen-binding fragment, comprising:
a1) a heavy-chain variable region (VH) comprising VH complementarity-determining region (CDR)1 of SEQ ID NO. 3, VH CDR2 of SEQ ID NO. 4, and VH CDR3 of SEQ ID NO. 5; and a light-chain variable region (VL) comprising VL CDR1 of SEQ ID NO. 6, VL CDR2 of a tripeptide sequence Lys-Ala-Ser, and VL CDR3 of SEQ ID NO. 7; or
a2) a heavy-chain variable region comprising VH CDR1 of SEQ ID NO. 8, VH CDR2 of SEQ ID NO. 9, and VH CDR3 of SEQ ID NO. 10; and a light-chain variable region comprising VL CDR1 of SEQ ID NO. 11, VL CDR2 of SEQ ID NO. 12, and VL CDR3 of SEQ ID NO. 13; or
a3) a heavy-chain variable region comprising VH CDR1 of SEQ ID NO. 14, VH CDR2 of SEQ ID NO. 15, and the VH CDR3 of SEQ ID NO. 10; and a light-chain variable region comprising the VL CDR1 of SEQ ID NO. 11, the VL CDR2 of SEQ ID NO. 12, and the VL CDR3 of SEQ ID NO. 13;
the Trop2-binding antibody is a polyclonal antibody or an anti-Trop2 monoclonal antibody; and
the Trop2 antigen-binding fragment is:
b1) a single-chain fragment variable (scFv); or
b2) a disulfide-stabilized Fv; or
b3) a fragment antigen-binding (Fab); or
b4) a fragment antigen-binding prime (F(ab')); or
b5) a monovalent fragment consisting of a VL domain, a VH domain, a light-chain constant region (CL) domain, and a heavy-chain constant region 1 (CH1) domain; or
b6) a fragment antigen-binding prime 2 (F(ab)₂), a bivalent fragment comprising two Fab fragments linked by a disulfide bond in a hinge region; or
b7) an Fv fragment consisting of a VL domain and a VH domain of a single arm of an antibody.

2. The Trop2-binding antibody or the Trop2 antigen-binding fragment according to claim 1, comprising: a heavy-chain variable region comprising the amino acid sequence of SEQ ID NO. 1 and a light-chain variable region comprising the amino acid sequence of SEQ ID NO. 2.

3. The Trop2-binding antibody or the Trop2 antigen-binding fragment according to claim 1, wherein the Trop2-binding antibody or the Trop2 antigen-binding fragment is a fully human antibody or a fully human antigen-binding fragment.

4. The Trop2-binding antibody or the Trop2 antigen-binding fragment according to claim 1, wherein the Trop2-binding antibody or the Trop2 antigen-binding fragment is a single-chain antibody.

5. A nucleic acid, wherein the nucleic acid encodes the Trop2-binding antibody or the Trop2 antigen-binding fragment according to any one of claims 1 to 4.

6. The nucleic acid according to claim 5, wherein the nucleic acid comprises a nucleotide sequence encoding the heavy-chain variable region comprising SEQ ID NO. 20 and a nucleotide sequence encoding the light-chain variable region comprising SEQ ID NO. 21.

7. A vector, wherein the vector comprises the nucleic acid according to claim 5 or 6.

8. A host cell, wherein the host cell comprises the nucleic acid according to claim 5 or 6 or a vector according to claim 7.

9. A method for producing an antibody or an antigen-binding fragment, comprising: culturing the host cell according to claim 8 and recovering the antibody or the antigen-binding fragment from a resulting culture.

10. An anti-Trop2 chimeric antigen receptor (CAR), wherein the anti-Trop2 CAR comprises anti-Trop2 scFv, wherein the anti-Trop2 scFv is the Trop2 antigen-binding fragment according to claim 1; and the Trop2 antigen-binding fragment is the scFv.

11. The anti-Trop2 CAR according to claim 10, wherein a structure of the anti-Trop2 CAR is cluster of differentiation (CD)8 leader - anti-Trop2 scFv - CD8 Hinge - CD8 transmembrane domain (TM) - costimulatory domain - intracellular signaling peptide, comprising a CD8 leader membrane receptor signal peptide, the anti-Trop2 scFv, a CD8 Hinge chimeric receptor hinge region, a CD8 TM chimeric receptor transmembrane domain, a costimulatory domain, and an intracellular signaling peptide that are linked in tandem sequentially, wherein the anti-Trop2 scFv is the Trop2 antigen-binding fragment according to claim 1; and the Trop2 antigen-binding fragment is the scFv.

12. An anti-Trop2 chimeric antigen receptor T-cell (CAR-T), an anti-Trop2 chimeric antigen receptor natural killer cell (CAR-NK), or an anti-Trop2 chimeric antigen receptor macrophage (CAR-M) modified with CAR, wherein the CAR is the anti-Trop2 CAR according to claim 10.

13. A use of the Trop2-binding antibody or the Trop2 antigen-binding fragment according to claim 1 or a nucleic acid according to claim 5 or a vector according to claim 7 or a host cell according to claim 8 or an anti-Trop2 CAR according to claim 10 or an anti-Trop2 CAR-T, an anti-Trop2 CAR-NK, or an anti-Trop2 CAR-M according to claim 12 in preparation of an anti-tumor drug.

14. The use according to claim 13, wherein the tumor is capable of expressing Trop2.
